(19)

Europäisches
Patentamt
European
Patent Office
Office européen
des brevets

(11) **EP 1 987 839 A1**

(12) **EUROPEAN PATENT APPLICATION**

(43) Date of publication:
 **05.11.2008 Bulletin 2008/45**

(51) Int Cl.:
 *A61K 39/00* (2006.01)   *C07K 16/28* (2006.01)

(21) Application number: **07290545.8**

(22) Date of filing: **30.04.2007**

(84) Designated Contracting States:
 **AT BE BG CH CY CZ DE DK EE ES FI FR GB GR HU IE IS IT LI LT LU LV MC MT NL PL PT RO SE SI SK TR**
 Designated Extension States:
 **AL BA HR MK RS**

(71) Applicants:
 • **I.N.S.E.R.M. Institut National de la Sante et de la Recherche Medicale**
  **75654 Paris Cedex 13 (FR)**
 • **Immutep**
  **91893 Orsay Cedex (FR)**

(72) Inventors:
 • **Triebel, Frédéric**
  **78000 Versailles (FR)**

 • **Vanhove, Bernard**
  **44400 Rezé (FR)**
 • **Haudebourg, Thomas**
  **44000 Nantes (FR)**

(74) Representative: **Bredema**
  **38, avenue de l'Opéra**
  **75002 Paris (FR)**

Remarks:
 The sequence listing, which is published as annex to the application documents, was filed after the date of filing. The applicant has declared that it does not include matter which goes beyond the content of the application as filed.

(54) **Cytotoxic anti-LAG-3 monoclonal antibody and its use in the treatment or prevention of organ transplant rejection and autoimmune disease**

(57)   The present invention concerns a molecule binding to LAG-3 protein and causing depletion of LAG-3+ activated T cells particularly said molecule is a cytotoxic anti-LAG-3 monoclonal antibody or fragment thereof. It also concerns a method of treating or preventing organ transplant rejection or autoimmune diseases in a mammal comprising administering to said mammal a therapeutically effective amount of said antibody.

EP 1 987 839 A1

**Description**

**I - Field of the invention**

The present invention is in the field of immunotherapy. More specifically, it relates to the treatment or prevention of organ transplant rejection or for treating autoimmune disease. The invention relates to molecule binding to LAG-3 protein and causing depletion of LAG-3[+] activated T cells. More specifically, it relates to cytotoxic LAG-3-specific monoclonal antibody or fragment thereof.

**II - Background**

Lymphocyte activation gene-3 (LAG-3, CD223) is up-regulated during the early stages of T-cell activation. The present invention is based on the analysis of the effects of cytotoxic antibodies against LAG-3 in acute cardiac allograft rejection (in vivo animal studies) and in in vitro experiments where selected LAG-3 monoclonal antibodies are efficient at low doses (<0.1 μg/ml) at depleting LAG-3[+] activated effector T cells.

Selectively depleting activated T lymphocytes might represent an immunosuppressive induction treatment able to result in the development of regulatory cells supporting a long-term survival of allogeneic organs in mice and primates (1). This has actually been demonstrated with anti-CD40L antibodies that deplete in vivo activated T cells through a Fc-dependent mechanism (2). However, anti-CD40L antibodies also target activated platelets in humans and affect the stability of arterial thrombi (3). Therefore the development of monoclonal antibodies to other molecules specific for T-cell activation has catalyzed attempts to achieve immunosuppression. One such molecule is LAG-3, which engages Class II on dendritic cells (DC) with a high affinity, enabling DC to become activated (4-6). The LAG-3 protein is expressed in vivo in activated CD4[+] and CD8[+] lymphocytes residing in inflamed secondary lymphoid organs or tissues but not in spleen, thymus or blood (7). In addition, LAG-3 can function as a negative regulator of activated human CD4 and CD8 T cells by inhibiting early events in primary activation (8).

**III - Summary of the invention**

The invention provides a molecule binding to LAG-3 protein and causing depletion of LAG-3[+] activated T cells. Said depletion can be measured by changes in peripheral blood lymphocyte numbers, in a tissue or an organ.

In a preferred embodiment the molecule binding to LAG-3 protein is a cytotoxic anti-LAG-3 monoclonal antibody or fragment thereof causing depletion of LAG-3+ activated T cells, said antibody comprising an Fc fragment from the human IgG1 or IgM (or mouse IgG2a) subclass and an Fab fragment which binds LAG-3 protein, said antibody exhibiting a complement-dependant cytotoxicity (CDC) and/or an antibody dependant cell cytotoxicity activity (ADCC).

The present invention further provides a method for treating or preventing organ transplant rejection or for treating autoimmune disease. Said method comprises the administration to a mammalian subject a therapeutically effective amount of a cytotoxic anti-LAG-3 monoclonal antibody or fragment thereof.

**IV - Description of the figures**

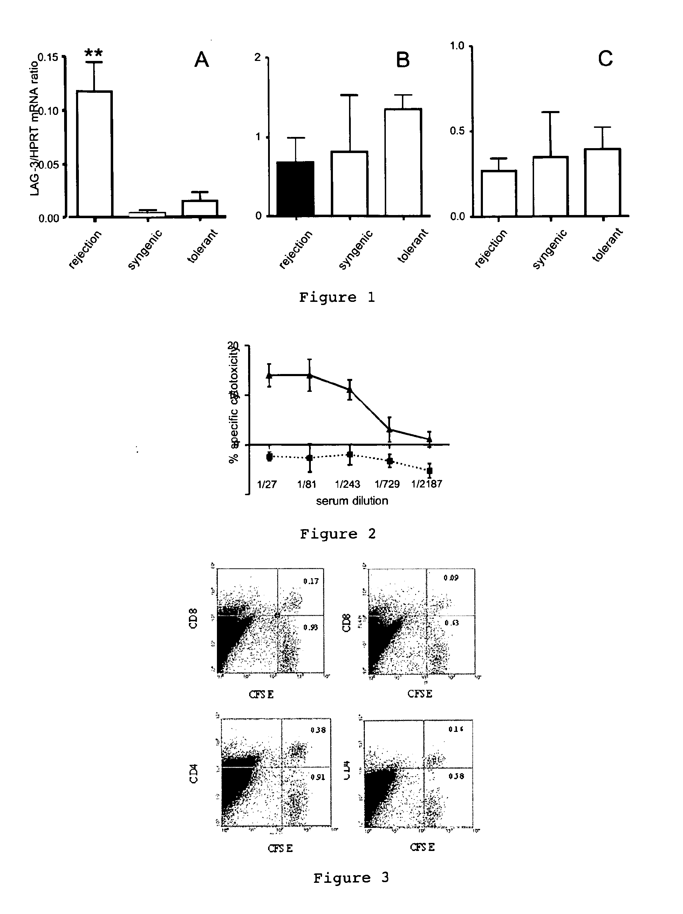

Figure 1: LAG-3 mRNA expression in cardiac allograft (A), in the spleen (B) and in lymph nodes (C). Expression of LAG-3 mRNA in heart grafts at day 5 was measured by quantitative RT-PCR and compared with housekeeping HPRT transcripts expression. Rejection: allograft without treatment. Syngenic: isograft. Tolerant: allograft in recipients receiving a tolerogenic (CsA+anti-CD28 antibodies) treatment. **: p<0.05 for syngenic and tolerant vs. rejection.

Figure 2: Characterization of anti-LAG-3 antibodies in a complement-dependant cytotoxicity assay. ConA-stimulated target cells were labeled with $^{51}$Cr and mixed with rabbit complement and anti-LAG-3 (full line) or preimmune (dotted line) serum at the indicated dilution. % cytotoxic activity is calculated as follows: (CPM of the assay - spontaneous CPM release)/ maximum released CPM obtained after cell lysis.

Figure 3: In vitro depleting activity of anti-LAG-3 antibodies. T cells from the spleen were activated for 48h with Con A to induce expression of LAG-3 and labeled with CFSE. 105 cells were injected i.v. to recipients that had been irradiated (4.5 Gy) 3 days before. Twenty-four hours after injection, recipients were sacrificed and the presence of CFSE[+] cells in the CD4[+] and CD8[+] compartments of the spleen analyzed by flow cytometry.

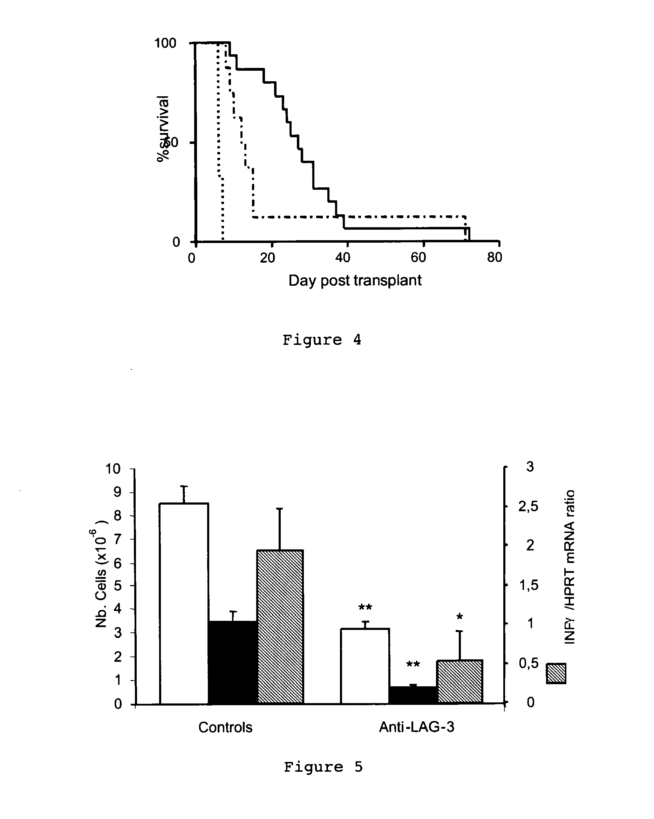

Figure 4: Heart graft survival after anti-LAG-3 antibodies administration. Lew.1A recipients of fully allogeneic (class I and II mismatch) Lew.1W hearts were treated by injections on days 0 and 3 of 200 $\mu$l (dashed line) or 600 $\mu$l (full line) rabbit anti-LAG-3 serum or of 600 $\mu$l pre-immune serum (dotted line). Graft survival was evaluated by daily evaluation of heartbeat. P<0.002 for 600 $\mu$l rabbit anti-LAG-3 serum vs. pre-immune serum.

Figure 5: Analysis of Graft infiltrating cells (GICs). GICs were extracted from cardiac allografts on day 5 from control-treated or anti-LAG-3 antibodies treated recipients. Cells were counted and analyzed by flow cytometry for the expression of LAG-3. White bars: total number of GICs. Black bars: LAG-3$^+$ GICs measured by flow cytometry (p< 0.01). Total RNA was also extracted from GICs and messenger for INF$\gamma$ were quantified by qPCR, relative to HPRT expression level (dashed bars; p< 0.05).

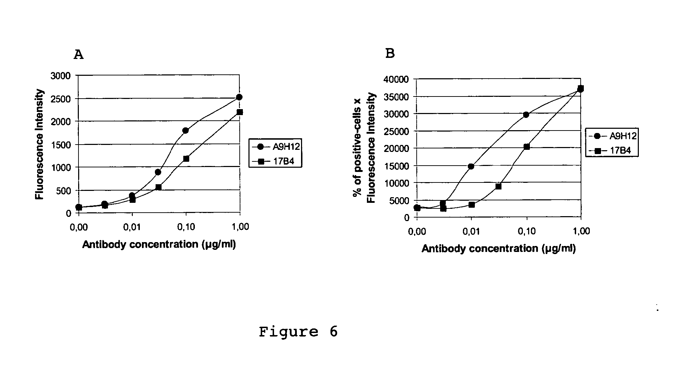

Figure 6: Comparison of A9H12 binding with the reference LAG-3 specific 17B4 mAb on LAG-3$^+$ CHO and activated human T cells.

A) *hLAG-3*-transfected CHO were dissociated from culture plastic using Versene buffer containing cation-chelating agent, incubated with indicated concentrations of A9H12 or 17B4 mAbs for 30 min at 4°C, washed and then incubated with a FITC-conjugated goat anti-mouse IgG+M (H+L) secondary antibody (5 $\mu$g/ml, Coulter) for 30 minutes at 4°C. After washing, cells were analysed by flow cytometry using a FACSCanto (BD Biosciences) and means of fluorescence intensity were plotted as a function of antibody concentration.
B) PBMCs from a healthy volunteer were stimulated for 2 days with SEB (1 $\mu$g/ml, Sigma Aldrich) to induce the expression of LAG-3 on T cells. PBMCs were stained as above. Data represent a weighted percentage, calculated as the percentage of LAG-3$^+$ cells in PBMCs x mean of fluorescence intensity of the LAG-3$^+$ cells, plotted as a function of antibody concentration.

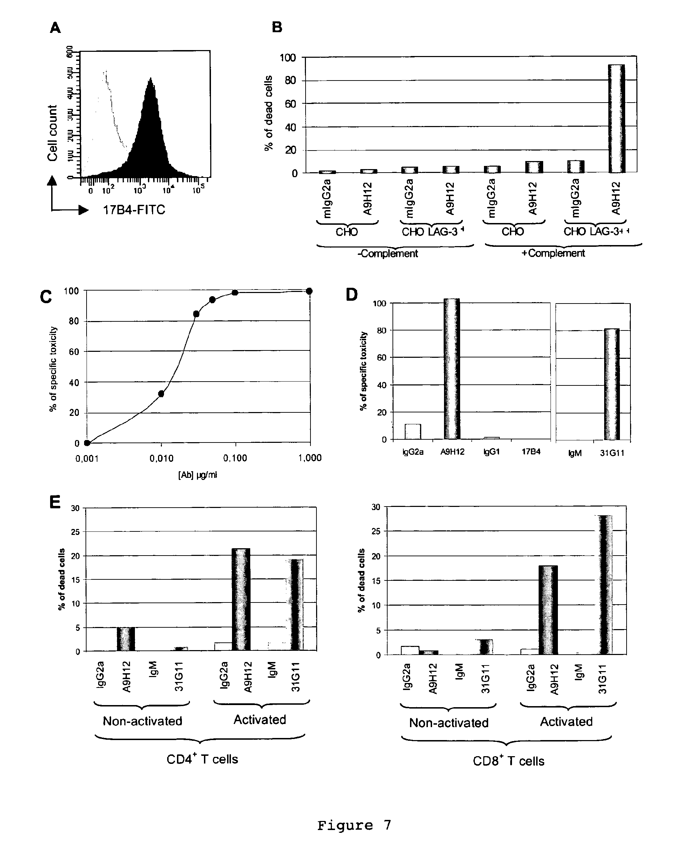

Figure 7: Complement-Dependent Cytotoxicity induced by A9H12 LAG-3 mAb.

A) *hLAG-3*-transfected and wild type CHO cells were labelled with FITC-conjugated anti-LAG-3 mAb (17B4) and the expression of LAG-3 on cell surface was analysed by flow cytometry using a FACSCanto. The histogram plots represent the mean fluorescence intensity of wt CHO (gray) and LAG-3$^+$ CHO (dark).
B) *hLAG*-3-transfected and wt CHO cells were washed in complete medium (MEM supplemented with 10 % heat inactivated Foetal Calf Serum, FCS) and incubated with 0.1 $\mu$g/ml of A9H12 LAG-3 mAb or mIgG2a isotype-control mAb (Southern Biotechnologies) in complete medium for 30 min at 4 °C. Cells were then washed and incubated in complete medium (-Complement) or in MEM supplemented with 10 % of freshly resuspended rabbit serum (Cerdalane Inc.) (+Complement) for 1 hour at 37°C. After washing, cells were stained with 7-AAD (Coulter Inc.) for 15 minutes at room temperature and immediately analysed by flow cytometry to determined the percentage of 7-AAD-positive cells corresponding to dead cells. Data represent the percentage of dead cells in each condition on *hLAG-3*-transfected and wt CHO cells as indicated.
C) LAG-3$^+$ CHO cells were incubated with indicated concentrations of A9H12 LAG-3 mAb for 30 min at 4°C and then incubated with MEM supplemented with 25 % rabbit serum for 1 hour at 37°C. After washing, cells were stained with 7-AAD (Coulter Inc.) and analysed by flow cytometry. The percentage of specific cytotoxicity is calculated according to the following formula

$$\frac{(\text{Sample Death} - \text{Spontaneous Death}) \times 100}{(\text{Maximal Death} - \text{Spontaneous Death})}$$

where Sample Death is the percentage of 7-AAD-positive cells in each condition, Spontaneous Death, the percentage of 7-AAD-positive cells without mAb and Maximal Death, the percentage of 7-AAD-positive cells with 10 $\mu$g/ml mAb.
D) LAG-3$^+$ CHO cells were incubated with 0.1 $\mu$g/ml A9H12, 17B4 or 31G11 LAG-3 mAb or with their corresponding isotype controls (IgG2a,IgG1 or IgM, respectively) for 30 min at 4°C and then incubated with MEM supplemented with 25 % rabbit serum for 1 hour at 37°C. Specific cytotoxicity was determined as above with a Maximal Death corresponding to 10 $\mu$g/ml A9H12 (left panel) and 0.1 $\mu$g/ml A9H12 (right panel).
E) PBMCs were stimulated with SEB (1 $\mu$g/ml) to induce LAG-3 expression on T cells and then used as target cells in the CDC assay in the presence of A9H12 or 31G11 LAG-3 mAb or their isotype controls. After staining cells with fluorochrome-conjugated CD3, CD4, CD8, CD25 and 17B4, the percentage of 7-AAD-positive cells was analysed on the indicated T cell subpopulations. Data represent the percentage of dead cells in each population (with spontaneous death in the absence of mAb being subtracted).

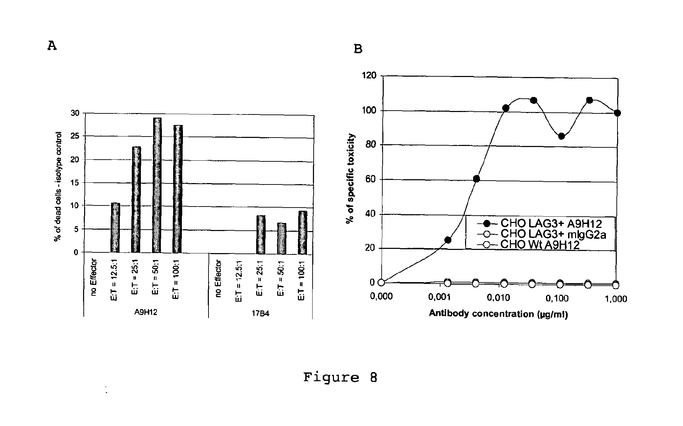

Figure 8: Antibody-Dependent Cell-mediated Cytotoxicity induced by A9H12 LAG-3 mAb

A) Effector cells (PBMCs) were stimulated with IL-2 (100 IU/ml, BD Biosciences) for 1 day. Target cells (hLAG-3-transfected CHO cells) were labelled with CFSE (a fluorescent vital dye) and incubated with 1 $\mu$g/ml A9H12, mIgG2a, 17B4 or mIgG1 for 20 min at room temperature. Effector cells and target cells were then mixed at indicated E:T ratios (E:T, Effector:Target) and incubated for 16 hours at 37°C. Non-adherent and adherent cells were harvested using Versene reagent, stained with 7-AAD and immediately analysed by flow cytometry to determine the percentage of 7-AAD-positive cells in the CFSE-positive population. Data represent the percentage of dead cells, with the non-specific cell death in the presence of the isotype control being subtracted.

B) CFSE-labelled wild-type or LAG-3$^+$ CHO target cells were incubated with indicated concentrations of A9H12 or mIgG2a and IL-2-stimulated PBMCs were added at a 50:1 E:T ratio and incubated for 16 hours at 37°C. Cell death was analysed as above and data represent the percentage of dead cells in CFSE-positive cells in the presence of A9H12 or its isotype-matched IgG2a control mAb.

## V - Detailed description

The present invention provides molecules binding to LAG-3 protein and causing depletion of LAG-3+ activated T cells. Said depletion can be measured by changes in peripheral blood lymphocyte numbers, a tissue or an organ.

The present invention relates preferably to human LAG-3 protein (hLAG-3 also named hereafter LAG-3). In a preferred embodiment the molecule binding to LAG-3 protein is a cytotoxic anti-LAG-3 monoclonal antibody or fragment thereof causing depletion of LAG-3+ activated T cells, said antibody comprising an Fc fragment from the human IgG1 or IgM (or mouse IgG2a) subclass and an Fab fragment which binds LAG-3 protein, said antibody exhibiting a complement-dependant cytotoxicity (CDC) and/or an antibody dependant-cell cytotoxicity activity (ADCC).

Lymphocyte activation gene-3 (LAG-3, CD223) is up-regulated during the early stages of T-cell activation. The present invention is based on the analysis of the effects of cytotoxic antibodies against LAG-3 in acute cardiac allograft rejection (*in vivo* animal studies) and in *in vitro* experiments where selected LAG-3 monoclonal antibodies are efficient at low doses (<0.1 $\mu$g/ml) at depleting LAG-3$^+$ activated effector T cells.

Fully vascularized heterotopic allogeneic heart transplantation was performed in rats across a full-MHC mismatch barrier (LEW.1W into LEW.1A). Recipients received two injections (day 0 and 3) of antibodies directed to the extraloop domain of LAG-3 or control antibodies. Graft survival, histology, mRNA transcripts and alloreactivity of lymphocytes were tested.

It was first noted that LAG-3 mRNA molecules accumulate in cardiac allografts undergoing rejection, but not in peripheral lymphoid organs. Administration of anti-LAG-3 antibodies inhibited graft infiltration by effectors mononuclear cells and prolonged allograft survival from 6 days in control antibodies-treated recipients to a median of 27 days.

It was found that cells expressing LAG-3 infiltrate rejected heart allografts and that targeting LAG-3 using cytotoxic antibodies as induction monotherapy delays acute rejection by reducing graft infiltration by T cells and monocytes.

Experiments showing that short courses of CD40L antibody therapy could achieve long-term graft survival in mice and primates have been initially interpreted as an effect of costimulation blockade. However, Monk *et al. (2)* showed that much of the efficacy of anti-CD40L therapy derives not from costimulation blockade, but from destruction of activated T cells. The outcome is a selective purging of potentially aggressive T cells that have experienced antigen.

Molecules that bind to LAG-3 protein and cause depletion of LAG-3+ activated T cells, according to the present invention, include antibodies (mono or polyclonal, preferably monoclonal) and fragment thereof, peptides and organic small molecules.

Cytotoxic anti-LAG-3 monoclonal antibody or fragment thereof according to the present invention causes depletion of more than 30% preferably more than 50 % of LAG-3+ activated T cells.

Cytotoxic anti-LAG-3 monoclonal antibody or fragment thereof according to the invention comprises antibodies with a murine IgG2a or a human IgG1 Fc region giving strong CDC or ADCC properties.

Cytotoxic anti-LAG-3 monoclonal antibody or fragment thereof according to the present invention exhibits (i) more than 50 % of maximal CDC activity at a mAb concentration of less than 0.1 $\mu$g/ml and/or (ii) more than 50 % of maximal ADCC activity at a mAb concentration of less than 0.1 $\mu$g/ml.

Molecules binding to LAG-3 protein and more particularly cytotoxic anti-LAG-3 monoclonal antibody, causing depletion of LAG-3+ activated T cells and antibody, can be produced by methods well known to those skilled in the art.

Antibodies generated against CD223 polypeptides can be obtained by direct injection of the CD223 polypeptides into an animal or by administering CD223 polypeptides to an animal, preferably a nonhuman The antibody so obtained will then bind the CD223 polypeptides itself. In this manner, even a sequence encoding only a fragment of the CD223 polypeptide can be used to generate antibodies binding the whole native CD223 polypeptide.

For preparation of monoclonal antibodies, any technique which provides antibodies produced by continuous cell line cultures can be used. Examples include the hybridoma technique (9), the trioma technique, the human B-cell hybridoma technique (10).

Techniques described for the production of single chain antibodies (US. Pat. No. 4,946,778) can be readily used to produce single chain antibodies to CD223 polypeptides. Also, transgenic mice may be used to express humanized antibodies to immunogenic CD223 polypeptides.

A first monoclonal antibody according to the present invention, called A9H12, is produced by the hybridoma deposited at the CNCM on April 27, 2007 under the access number CNCM I-3755.

A second monoclonal antibody according to the present invention, called 31G11, is produced by the hybridoma deposited at the CNCM on April 27, 2007 under the access number CNCM I-3756.

The invention is also directed to the use of a cytotoxic anti-LAG-3 monoclonal antibody or fragment thereof for the manufacture of a medicament for treating or preventing organ transplant rejection or for treating autoimmune disease.

The present invention further provides a method for treating or preventing organ transplant rejection or for treating autoimmune disease. Said method comprises the administration to a mammalian subject a therapeutically effective amount of a cytotoxic anti-LAG-3 monoclonal antibody or fragment thereof.

Organ transplant rejection refers to the graft of an organ in an allogenic host. It may be useful for treating organisms suffering from conditions resulting in an abnormally high T-cell population or deleterious T-cell activity, for example graft rejection mediated by host T-cells, graft vs. host disease and T-cell mediated autoimmune and inflammatory diseases such as rheumatoid arthritis, type 1 diabetes, muscular sclerosis, etc. The methods of the invention may be applied to any organism which contains T-cells that express CD223. This includes, but is not limited to, any mammal and particularly includes humans and mice.

Auto-immune deseases are diseases in which the subject's own immune system reacts against the subject cells. Auto-immune disease which are amenable to treatments according to the present invention include autoimmune hemolytic anemia, autoimmune thrombocytopenia purpura, Goodpasture's syndrome, pemphigus vulgaris, acute rheumatic fever, mixed essential cryoglobulinemia, systemic lupus erythematosus, insulin-dependent diabetes mellitus, theumatoid arthritis, Grave's disease, Hashimoto's thyroiditis, myasthenia gravis, and multiple sclerosis.

A method for depleting LAG-3+ activated T cells from a sample from a patient according to the present invention comprises reacting the sample with an antibody composition comprising an antibody described above.

A pharmaceutical composition according to the present invention comprises from 30 to 300 mg per dose of a cytotoxic monoclonal antibody described above and one or more pharmaceutically acceptable carriers and/or diluents for administration to a mammal. The pharmaceutical composition of the present invention may be specially formulated for administration in solid or liquid form.

EXAMPLE 1 : LAG-3-positive cells targeted with cytotoxic antibodies

Material and methods

*Animals and transplantations*

Eight- to 12-week-old male Lewis.1W (LEW.1W, haplotype RT1$^u$) and Lewis.1A (LEW. 1A, haplotype RT1$^a$) congeneic rats (Centre d'Elevage Janvier, Le Genest-Saint-Isle, France), differed in their entire MHC region. Heterotopic LEW.1W heart transplantation was performed as previously described (11). Graft survival was evaluated by palpation through the abdominal wall.

*Anti-LAG-3 antibodies*

A synthetic peptide corresponding to the extraloop domain of the rat LAG-3 protein (NCBI accession nb DQ438937; peptide DQPASIPALDLLQGMPSTRRHPPHR) was linked to ovalbumin and used to immunise two rabbits. Pre-immune and immune sera, collected on day 63 after the 4$^{th}$ immunisation, were assayed by ELISA on immunogen and peptide and by flow cytometry on Con-A activated rat spleen cells. Pre-immune sera were negative in both assays. Pooled immune sera presented a titer (dilution for 50% signal) of 1/60000 by ELISA and of 1/1000 by FACS, and presented a specificity for activated T cells.

*Complement-dependant cytotoxicity assay*

Complement-mediated antibody-dependent cytotoxicity was tested using rabbit sera against Lewis 1A T cells in a $^{51}$Cr release assay. A total of $2\times10^6$ Lewis 1A T cells were labelled with 30 $\mu$Ci of $^{51}$Cr for 90 min at 37°C in RPMI (GIBCO) with 10% FCS. After three washes, T cells were distributed in 96 V-bottomed plates and incubated with rabbit complement and serial dilutions of heat-inactivated rabbit serum. After 4 h at 37°C, $^{51}$Cr release was measured in the supernatants using a scintillation counter. Specific cytotoxicity was calculated according to the following formula: (experimental release - spontaneous release) x 100/(maximum release - spontaneous release).

*In vivo antibody-induced cytotoxicity*

Cytotoxic activity of anti-LAG-3 antibodies against LAG-3+ cells was evaluated in vivo. ConA-activated (48h) LEW.1W splenocytes were labelled with the CFSE and transferred ($10^8$ cells) into irradiated (4.5 Gy, day -3) LEW.1A recipients, together with anti-LAG-3 antibodies. On day 1, recipients were sacrificed and the presence of CFSE-positive cells evaluated by flow cytometry in lymphoid organs and in the blood.

*Immunostaining*

Graft samples were embedded in Tissue Tek (OCT Compound, Torrance, CA, USA), snap-frozen in liquid nitrogen, cut into 5 $\mu$m sections and fixed in acetone. Endogenous biotin activity was blocked using the Dako biotin blocking system (Dako, Trappes, France). Sections were then labelled by a three-step indirect immunoperoxidase revelation. The area of each immunoperoxidase-labeled tissue section infiltrated by cells was determined by quantitative morphometric analysis. Positively stained cells on each slide were counted by morphometric analysis using point counting analysis (14) with a 121-intersection squared grid in the eyepiece of the microscope. Briefly, the percentage of the area of each graft section occupied by cells of a particular antigenic specificity (area infiltrate) was calculated as follows: [number of positive cells under grid intersection/(total number of grid intersections = 121)] x 100. The graft sections were examined at a magnification of x400. The accuracy of the technique is proportional to the number of points counted. Thus, to maintain a SE of <10%, 15 fields were counted for each labeled section. Results are expressed as the percentage of the area of the tissue section infiltrated by leukocytes (determined with OX1, OX30 labeling) and the phenotypic composition of the infiltrate and subpopulations which are related to the percentage of total leukocytes and are expressed as the percentage of leukocytes.

*Graft_infiltrating cell extraction staining*

Dilacerated hearts were digested with collagenase D (2 mg/ml; Boehringer Mannheim) for 10 min at 37°C. Cells were then collected by extraction through a stainless steel mesh. The resulting suspension was then clarified by Ficoll isolation.

*Quantitative RT-PCR*

Real-time quantitative PCR was performed in an Applied Biosystems GenAmp 7700 Sequence Detection System using SYBR Green PCR Core Reagents (Applied Biosystems, Foster City, CA). The following oligonucleotides were used in this study: rat LAG-3: upper primer is 5'-ATATGAATTCACAGAGGAGATGAGGCAG-3' and lower primer is 5'-ATATGAATTCTCCTGGTCAGAGCTGCCT-3'. Rat INF-g: upper primer is 5'-TGGATGCTATGGAAGGAAAGA-3' and lower primer is 5'-GATTCTGGTGACAGCTGGTG-3'. Rat HPRT: upper primer is 5'-CCTTGGTCAAGCAGTACAGCC-3' and lower primer is 5'-TTCGCTGATGACACAAACATGA-3'. A constant amount of cDNA corresponding to the reverse transcription of 100 ng of total RNA was amplified in 25 $\mu$l of PCR mix containing 300 nM of each primer; 200 $\mu$M dATP, dGTP, dCTP; 400 $\mu$M dUTP; 3 mM MgCl$_2$; 0.25 U of uracyl-N-glycosylase; 0.625 U of AmpliTaq Gold DNA polymerase. The mix was subjected to 40 cycles of amplification. The real-time PCR data were plotted as the $\Delta R_n$ fluorescence signal vs. the cycle number. The $\Delta R_n$ values were calculated by the Applied Biostystems 7700 sequence detection software using the formula: $\Delta R_n = (R^+_n) - (R^-_n)$, where $R^+_n$ is the fluorescence signal of the product at any given time, $R_n^-$ is the mean fluorescence signal during cycles 3-13 and referred to as the baseline. The $C_t$ value is defined as the cycle number at which the $\Delta R_n$ crosses a threshold. The threshold is set above the background fluorescence to intersect the exponential portion of the amplification curve of a positive reaction. The Ct is inversely proportional to the log amount of template within the PCR.

*Statistical analyses*

Statistical significance was evaluated using aa Mann-Whitney test for the comparison of two groups. Graft survival was evaluated by Kaplan-Meier analysis using the log rank test.

<u>Results</u>

*LAG-3 mRNA expression in rejected allograft and lymphoid organs*

LAG-3 is expressed by activated T cells in inflamed lymphoid organs and tissues (7). In order to see if LAG-3 is also expressed in rejected allografts, hearts grafts from LEW.1W to LEW.1A rat recipients were analyzed on day 5 (rejection occurring on day 6). Messenger RNA for LAG-3 was analyzed and compared with allografts receiving a tolerance-inducing regiment (anti-CD28 antibodies + CSA, as described *(16))* and with isografts. Rejected allografts presented a 7-fold and a 25-fold accumulation of LAG-3 mRNA as compared with tolerated and syngeneic grafts, respectively (Fig. 1A). Such an accumulation was not detected in lymph nodes (Fig. 1B) or in the spleen of rejecting recipients (Fig. 1C).

*Mechanism of action of anti-LAG-3 antibodies*

Anti-LAG-3 antibodies were produced in rabbits by immunization with a synthetic peptide from the extraloop of LAG-3 Ig-like N-terminal domain , involved in the interaction of LAG-3 with Class II (ref PNAS Huard 1997). Post-immune serum, as well as the IgG fraction, stained <1% of rat spleen cells and 40% of rat spleen cells activated for 48h with ConA, PMA + ionomycin or PHA. Pre-immune serum was negative (data not shown). In order to characterize the effect of anti-LAG-3 antibodies on LAG-3$^+$ cells, complement and ADCC-dependant cytotoxicity was assayed in vitro. Fifteen % of ConA-activated spleen cells were lysed in the complement-dependant cytotoxicity assay (Fig. 2). Given that only 40% of the ConA-activated target cells expressed LAG-3, this assay revealed that about 37 % of the LAG-3$^+$ spleen cells present in the preparation were lysed in vitro as a result of complement activation.
*In vivo,* the depleting activity of anti-LAG-3 antibodies was estimated by measuring the fate of CFSE-labeled activated T cells adoptively transferred to irradiated rat recipients. One day after the injection of therapeutic doses of anti-LAG-3 immune serum, only half the amount of CFSE$^+$/CD4$^+$ and CFSE$^+$/CD8$^+$ cells could be recovered from the spleen, as compared with similar injections of pre-immune serum (Fig. 3).

*Anti-LAG-3 antibodies delay heart allograft rejection*

From preliminary pharmacokinetic observations, we established that two i.v. injections of 600 µl of anti-LAG-3 rabbit serum on days 0 and 3 resulted in the maintenance of anti-LAG-3 binding activity in recipient's serum for at least 2 weeks. This treatment delayed cardiac allograft rejection from 6 days in untreated and control-treated recipients to a median of 27 days. All recipients, however, eventually rejected their graft within 10 weeks (Fig. 4). On day 5, grafts from control-treated recipients were heavily infiltrated by activated T cells and this infiltrate was less important in anti-LAG-3 treated recipients. Infiltration by CD25$^+$ cells and NK cells, however, was not modified by the treatment. Since our anti-LAG-3 antibodies do not recognize LAG-3 in immunohistology, LAG-3 expression by graft infiltrating cells (GICs) was analyzed by flow cytometry after extraction. An average of 8.5 10$^6$ $\pm$ 0.76 GICs could be recovered from control rejected grafts. From heart allografts from anti-LAG-3 treated recipients, only 3.16 $\pm$0.44 10$^6$ GICs could be recovered (n=3; p< 0.005). GICs contained 41.17 $\pm$ 1 % LAG-3$^+$ cells in controls (i.e. 3.5 10$^6$ cells) versus 22.2 $\pm$ 0.9 % in treated animals (i.e. 0.7 10$^6$ cells; n=3; p<0.0005; Fig. 5). Analysis of mRNA transcript reinforced these observations that infiltration of heart graft by mononuclear cells was reduced since we measured four times less INFγ mRNA molecules in treated grafts (Fig. 5).

*Anti-LAG-3 antibodies inhibit ongoing acute heart allograft delay rejection*

In order to investigate whether anti-LAG-3 antibodies might serve as a treatment of an ongoing acute allograft rejection, we grafted LEW.1W hearts into LEW.1A allogenic recipients that we maintained untreated over 3 or 4 days. At that time, recipients received an injection of 600 microliter of control or anti-LAG-3 rabbit serum. Control-treated recipients rejected the allografts on day 5 whereas anti-LAG-3 antibodies-treated recipients rejected only on day 11 (Table 1).

Table 1

| Treatment | Day of rejection | Median survival |
|---|---|---|
| Control serum on day 3 | 5, 5, 5 | 5 |
| Control serum on day 4 | 5, 5, 5 | 5 |
| Anti-LAG-3 serum on day 3 | 12, 13, 9 | 12 (p<0.05 vs. control) |

(continued)

| Treatment | Day of rejection | Median survival |
|---|---|---|
| Anti-LAG-3 serum on day 4 | 10, 13, 13, 19 | 12.5 (p<0.05 vs. control) |

Table 1: Heart graft recipients were treated on day 3 or 4 with control or anti-LAG-3 antibodies. Rejection was monitored by daily heart palpation.

EXAMPLE 2: Generation of new high affinity hLAG-3 mAbs

Material and methods

Mice were immunized 3 times with hLAG-3-transfected CHO cells ($10^7$ cells, intra-peritoneal injection), followed by a boost *i.v.* injection with 10 $\mu$g IMP321, the clinical-grade hLAG-3Ig recombinant protein. Three days after the boost, splenocytes were fused with the X63.AG8653 fusion partner to yield hybridoma cells. Supernatants from hybridomas were screened for their specific binding (FACS analysis) on hLAG-3-tranfected CHO versus wild type (wt) CHO cells.

One murine IgG2a antibody (580.1E9H3A9H12, called A9H12) was selected, subcloned to yield a stable cell line and further characterized for its potency to deplete LAG-3$^+$ cells through CDC (complement-dependent cytotoxicity) and ADCC (antibody-dependent cell-mediated cytotoxicity), given that the murine IgG2a Fc region is known to be the most efficient Fc isotype in mice at delivering these activities, even on heterologous cells (i.e. CHO cells or human PBMCs). Similarly, a second IgM antibody (31G11E8, called 31G11) was also selected.

Results

Dose-dependent binding of A9H12 was first compared to the reference LAG-3-specific 17B4 mAb on hLAG-3-tranfected CHO cells and on LAG-3$^+$ *in vitro* activated human T cells (Figure 6). A9H12 displayed a greater binding than the reference 17B4 mAb on both cell types. For instance, significant binding of A9H12 to activated human T cells was observed with a concentration as low as 0.01 $\mu$g/ml.

For CDC testing, the target cells used in this assay were LAG-3$^+$ CHO cells compared to wt CHO cells (Figure 7A). Both types of cells were incubated for 1 hour at 37°C with either A9H12, its murine isotype-matched IgG2a negative control mAb, 31G11, its murine isotype-matched IgM negative control or the reference 17B4 (IgG1) mAb and rabbit serum containing active complement. Cell viability was then assessed using 7-Amino-Actinomycin D (7-AAD), a fluorescent dye labelling cells which have lost their membranous integrity, a phenomenon which appeared rapidly after death. The percentage of 7-AAD-positive CHO cells (i.e. dead target cells) was determined by flow cytometry analysis. A9H12 displayed a potent and specific cytotoxic activity in this CDC assay, killing only LAG-3$^+$ CHO cells in the presence of complement (Figure 7B). The anti-LAG-3 Ab was titered down to determined the efficacy of the antibody to activate CDC at low concentration of antibody. A9H12 efficiently induced LAG-3$^+$ CHO cells killing at a concentration as low as 0.01 $\mu$g/ml (Figure 7C). The IgG1 17B4 antibody was also tested in this assay and had no effect (Figure 2D, left panel), showing that not all LAG-3 mAbs could induce cytotoxicity in this bioassay. As observed with A9H12, the second 31G11 LAG-3-specific mAb also induced LAG-3$^+$ CHO cells killing (Figure 7D, right panel).

The CDC bioassay was also performed on PBMCs stimulated with the superantigen SEB. The cytotoxicity of A9H12 and 31G11 were analysed on both activated (namely CD25$^+$/LAG-3$^+$ cells) and non-activated (namely CD25$^-$/LAG-3$^-$ cells) CD4$^+$ helper T and CD8$^+$ cytotoxic T cells. Only activated CD4$^+$ and CD8$^+$ T cells were specifically killed by A9H12 and 31G11 (Figure 7E), showing that activated human T cells are susceptible to A9H12- or 31G11-specific killing in the presence of complement.

For ADCC testing, PMBCs were stimulated for one day with IL-2 to serve as effector cells and LAG-3$^+$ CHO cells were labelled with the vital dye CFSE to serve as target cells. In the presence of A9H12, PBMCs were able to kill a significant percentage of LAG-3$^+$ CHO cells and this effect was increased with the number of effector cells (Figure 8A). In the presence of 17B4, only a small percentage of target cells was killed even at a high E:T ratio (Figure 8A), showing that not all LAG-3 mAbs could induce cytotoxicity in this bioassay. The A9H12 LAG-3 mAb was titered down to determine the efficacy of the antibody to induce ADCC at low concentration of antibody. A9H12 efficiently induced LAG-3$^+$ CHO cells killing at a concentration as low as 0.01 $\mu$g/ml (Figure 8B).

**References**

1. Waldmann H. The new immunosuppression: just kill the T cell. Nat Med 2003; 9 (10): 1259.

2. Monk NJ, Hargreaves RE, Marsh JE, et al. Fc-dependent depletion of activated T cells occurs through CD40L-specific antibody rather than costimulation blockade. Nat Med 2003; 9 (10): 1275.

3. Andre P, Prasad KS, Denis CV, et al. CD40L stabilizes arterial thrombi by a beta3 integrin--dependent mechanism. Nat Med 2002; 8 (3): 247.

4. Avice MN, Sarfati M, Triebel F, Delespesse G, Demeure CE. Lymphocyte activation gene-3, a MHC class II ligand expressed on activated T cells, stimulates TNF-alpha and IL-12 production by monocytes and dendritic cells. J. Immunol. 1999; 162: 2748.

5. Andreae S, Piras F, Burdin N, Triebel F. Maturation and activation of dendritic cells induced by lymphocyte activation gene-3 (CD223). J Immunol 2002; 168 (8): 3874.

6. Andreae S, Buisson S, Triebel F. MHC class II signal transduction in human dendritic cells induced by a natural ligand, the LAG-3 protein (CD223). Blood 2003; 102 (6): 2130.

7. Triebel F. LAG-3: a regulator of T-cell and DC responses and its use in therapeutic vaccination. Trends Immunol 2003; 24 (12): 619.

8. Macon-Lemaitre L, Triebel F. The negative regulatory function of the lymphocyte-activation gene-3 coreceptor (CD223) on human T cells. Immunology 2005; 115 (2): 170.

9. Kohler and Milstein. Continuous cultures of fused cells secreting antibody of predefined specificity. Nature. 1975 256(5517):495-71975, Nature, 256:495-497.

10. Kozbor et al., 1985, in Monoclonal Antibodies and Cancer Therapy, Alan R. Liss, Inc., pp. 77-96.

11. Ono et al, Improved technique of heart transplantation in rats. J. Thorac. Cardiovasc. Surg. 57, 225-229.

**Claims**

1. A molecule binding to LAG-3 protein and causing depletion of LAG-3$^+$ activated T cells.

2. A molecule binding to LAG-3 protein according to claim 1 wherein said molecule is a cytotoxic anti-LAG-3 monoclonal antibody or fragment thereof causing depletion of LAG-3$^+$ activated T cells measured by changes in peripheral blood lymphocyte numbers, said antibody comprising the human IgG1 or IgM or murine IgG2a subclass Fc fragment and an Fab fragment which binds LAG-3 protein,
   wherein said antibody exhibits a complement-dependent cytotoxicity (CDC) and/or an antibody-dependent cell cytotoxicity activity (ADCC).

3. A cytotoxic anti-LAG-3 monoclonal antibody or fragment thereof according to claim 2, wherein said monoclonal antibody causes depletion of more than 30% preferably more than 50 % of LAG-3$^+$ activated T cells.

4. A cytotoxic anti-LAG-3 monoclonal antibody or fragment thereof according to any one of claims 2 or 3,
   wherein said monoclonal antibody exhibits (i) more than 50 % of maximal CDC activity at a mAb concentration of less than 0.1 $\mu$g/ml or (ii) more than 50 % of maximal ADCC activity at a mAb concentration of less than 0.1 $\mu$g/ml.

5. A cytotoxic anti-LAG-3 monoclonal antibody according to any one of claims 2 to 4, produced by the hybridoma deposited at the CNCM on April 27, 2007 under the access number CNCM I-3755 or a fragment thereof.

6. A cytotoxic anti-LAG-3 monoclonal antibody according to any one of claims 2 to 4, produced by the hybridoma deposited at the CNCM on April 27, 2007 under the access number CNCM I-3756 or a fragment thereof.

7. Use of a cytotoxic anti-LAG-3 monoclonal antibody or fragment thereof according to any one of claims 2 to 6 for the manufacture of a medicament for treating or preventing organ transplant rejection or for treating autoimmune diseases.

8. A method of treating or preventing organ transplant rejection or autoimmune diseases in a mammal comprising administering to said mammal a therapeutically effective amount of an antibody according to any one of claims 2 to 6.

9. A method for depleting LAG-3$^+$ activated T cells from a sample from a patient comprising reacting the sample with an antibody composition comprising an antibody according to to any one of claims 2 to 6.

10. A pharmaceutical composition comprising from 30 to 300 mg per dose of a cytotoxic monoclonal antibody according to any one of claims 2 to 6 and a pharmaceutically acceptable carrier and/or diluents for administration to a mammal.

Figure 1

Figure 2

Figure 3

Figure 4

Figure 5

A

B

Figure 6

Figure 7

A

B

Figure 8

**European Patent**
**Office**

## PARTIAL EUROPEAN SEARCH REPORT

Application Number

which under Rule 45 of the European Patent Convention shall be considered, for the purposes of subsequent proceedings, as the European search report

EP 07 29 0545

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MACON-LEMAITRE LAETITIA ET AL: "The negative regulatory function of the lymphocyte-activation gene-3 coreceptor (CD223) on human T cells" IMMUNOLOGY, vol. 115, no. 2, June 2005 (2005-06), pages 170-178, XP002453818 ISSN: 0019-2805 | 1-4 | INV. A61K39/00 C07K16/28 |
| Y | * the whole document * | 7,8,10 | |
| X | US 5 976 877 A (HERCEND THIERRY [FR] ET AL) 2 November 1999 (1999-11-02) | 1 | |
| Y | * claims * | 2-6 | |
| X | WO 2004/078928 A (PARDOLL DREW M [US]; HUANG CHING-TAI [US]; VIGNALI DARIO A [US]; WORKM) 16 September 2004 (2004-09-16) * claims 49,50 * | 1,9 | |
| | -/-- | | |

TECHNICAL FIELDS
SEARCHED (IPC)

C07K
A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 5 October 2007 | Sommerfeld, Teresa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**

## PARTIAL EUROPEAN SEARCH REPORT

**Application Number**

EP 07 29 0545

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| Y | MONK NICOLA J ET AL: "Fc-dependent depletion of activated T cells occurs through CD40L-specific antibody rather than costimulation blockade." NATURE MEDICINE, vol. 9, no. 10, October 2003 (2003-10), pages 1275-1280, XP002453819 ISSN: 1078-8956 * the whole document * ----- | 2-8,10 | |
| | | | **TECHNICAL FIELDS SEARCHED (IPC)** |

EPO FORM 1503 03.82 (P04C10)

Although claim 8 is directed to a method of treatment of the human/animal body (Article 52(4) EPC), the search has been carried out and based on the alleged effects of the compound/composition.

-----

## ANNEX TO THE EUROPEAN SEARCH REPORT
## ON EUROPEAN PATENT APPLICATION NO.

EP 07 29 0545

This annex lists the patent family members relating to the patent documents cited in  the above-mentioned European search report.
The members are as contained in the European Patent Office EDP file on
The European Patent Office is in no way liable for these particulars which are merely given for the purpose of information.

05-10-2007

| Patent document cited in search report | | Publication date | Patent family member(s) | | Publication date |
|---|---|---|---|---|---|
| US 5976877 | A | 02-11-1999 | NONE | | |
| WO 2004078928 | A | 16-09-2004 | AU | 2004217526 A1 | 16-09-2004 |
| | | | CA | 2517287 A1 | 16-09-2004 |
| | | | EP | 1596871 A2 | 23-11-2005 |
| | | | JP | 2006523226 T | 12-10-2006 |

EPO FORM P0459

For more details about this annex : see Official Journal of the European Patent Office, No. 12/82

REFERENCES CITED IN THE DESCRIPTION

**Patent documents cited in the description**

- US 4946778 A

**Non-patent literature cited in the description**

- **WALDMANN H.** The new immunosuppression: just kill the T cell. *Nat Med,* 2003, vol. 9 (10), 1259
- **MONK NJ ; HARGREAVES RE ; MARSH JE et al.** Fc-dependent depletion of activated T cells occurs through CD40L-specific antibody rather than costimulation blockade. *Nat Med,* 2003, vol. 9 (10), 1275
- **ANDRE P ; PRASAD KS ; DENIS CV et al.** CD40L stabilizes arterial thrombi by a beta3 integrin--dependent mechanism. *Nat Med,* 2002, vol. 8 (3), 247
- **AVICE MN ; SARFATI M ; TRIEBEL F ; DE-LESPESSE G ; DEMEURE CE.** Lymphocyte activation gene-3, a MHC class II ligand expressed on activated T cells, stimulates TNF-alpha and IL-12 production by monocytes and dendritic cells. *J. Immunol.,* 1999, vol. 162, 2748
- **ANDREAE S ; PIRAS F ; BURDIN N ; TRIEBEL F.** Maturation and activation of dendritic cells induced by lymphocyte activation gene-3 (CD223. *J Immunol,* 2002, vol. 168 (8), 3874
- **ANDREAE S ; BUISSON S ; TRIEBEL F.** MHC class II signal transduction in human dendritic cells induced by a natural ligand, the LAG-3 protein (CD223. *Blood,* 2003, vol. 102 (6), 2130
- **TRIEBEL F.** LAG-3: a regulator of T-cell and DC responses and its use in therapeutic vaccination. *Trends Immunol,* 2003, vol. 24 (12), 619
- **MACON-LEMAITRE L ; TRIEBEL F.** The negative regulatory function of the lymphocyte-activation gene-3 coreceptor (CD223) on human T cells. *Immunology,* 2005, vol. 115 (2), 170
- **KOHLER ; MILSTEIN.** Continuous cultures of fused cells secreting antibody of predefined specificity. *Nature,* 1975, vol. 256 (5517), 495-71975
- *Nature,* vol. 256, 495-497
- **KOZBOR et al.** Monoclonal Antibodies and Cancer Therapy. Alan R. Liss, Inc, 1985, 77-96
- **ONO et al.** Improved technique of heart transplantation in rats. *J. Thorac. Cardiovasc. Surg.,* vol. 57, 225-229